# EUROPEAN PATENT SPECIFICATION

(11) **EP 1 987 354 B1**
(45) Date of publication and mention of the grant of the patent: **03.11.2010**
(21) Application number: 07706053.1
(22) Date of filing: 28.01.2007
(51) Int. Cl.: G01N 33/49, G01N 33/86

(54) **METHODS FOR DETERMINING BLOOD COAGULATION**
VERFAHREN ZUR BLUTGERINNUNGSBESTIMMUNG
PROCÉDÉS POUR ÉVALUER LA COAGULATION SANGUINE

(30) Priority: 27.01.2006 US 762519 P
(43) Date of publication of application: 05.11.2008
(73) Proprietor: Rappaport Family Institute for Research in the Medical Sciences, Haifa 31096 (IL)
(72) Inventor: BRENNER, Benjamin, 34788 Haifa (IL); AHARON, Anat, 36066 Kiryat-tivon (IL)
(74) Representative: Fichter, Robert Arno
(86) International application number: PCT/IL2007/000110
(87) International publication number: WO 2007/086069

(56) References cited:
- AHARON ANAT ET AL: "Tissue factor and tissue factor pathway inhibitor levels in trophoblast cells: implications for placental hemostasis." THROMBOSIS AND HAEMOSTASIS OCT 2004, vol. 92, no. 4, October 2004 (2004-10), pages 776-786, XP002432337 ISSN: 0340-6245
- AHARON A ET AL: "Placental TFPI is decreased in gestational vascular complications and can be restored by maternal enoxaparin treatment [6]" JOURNAL OF THROMBOSIS AND HAEMOSTASIS 2005 UNITED KINGDOM, vol. 3, no. 10, 2005, pages 2355-2357, XP002432338 ISSN: 1538-7933 1538-7836
- SHIMURA MINORI ET AL: "Plasma tissue factor and tissue factor pathway inhibitor levels in patients with disseminated intravascular coagulation" AMERICAN JOURNAL OF HEMATOLOGY, vol. 52, no. 3, 1996, pages 165-170, XP002432339 ISSN: 0361-8609
- SAIGO M ET AL: "Imbalance of plasminogen activator inhibitor-I/ tissue plasminogen activator and tissue factor/tissue factor pathway inhibitor in young Japanese men with myocardial infarction" THROMBOSIS AND HAEMOSTASIS 2001 GERMANY, vol. 86, no. 5, 2001, pages 1197-1203, XP008078391 ISSN: 0340-6245
- SHET ARUN S ET AL: "Sickle blood contains tissue factor-positive microparticles derived from endothelial cells and monocytes." BLOOD, vol. 102, no. 7, 1 October 2003 (2003-10-01), pages 2678-2683, XP002432340 ISSN: 0006-4971
- SIMAK ET AL: "Cell Membrane Microparticles in Blood and Blood Products: Potentially Pathogenic Agents and Diagnostic Markers" TRANSFUSION MEDICINE REVIEWS, GRUNE AND STRATTON, ORLANDO, FL,, US, vol. 20, no. 1, January 2006 (2006-01), pages 1-26, XP005210302 ISSN: 0887-7963
- MOREL ET AL: "Microparticules circulantes au cours des traumatismes graves et des sepsis : un element du couplage inflammation-thrombose" ANNALES FRANCAISES D'ANESTHESIE ET DE REANIMATION, MASSON, PARIS, FR, vol. 25, no. 9, September 2006 (2006-09), pages 955-966, XP005658304 ISSN: 0750-7658
- AHARON ANAT ET AL: "Microparticles induce procoagulant and apoptotic effects on endothelial cells." BLOOD, vol. 108, no. 11, Part 2, November 2006 (2006-11), page 60B, XP002432341 & SYMPOSIUM OF THE INTERNATIONAL-SOCIETY-OF-MOLECULAR-EVOLUTI ON; GUANANACASTE, COSTA RICA; JANUARY 08 -12, 2001 ISSN: 0006-4971

## Description

### FIELD AND BACKGROUND OF THE INVENTION

The present invention relates to a method of determining blood coagulation.

Changes in the coagulation balance, due to procoagulant microparticles, is associated with hereditary or acquired thrombophilia, inflammatory complications, acute coronary syndromes and diseases such as diabetes mellitus and cancer. Hyper-coagulation predicts elevated risk for a thrombotic event, while hypocoagulation is associated with a bleeding tendency.

The procoagulant microparticles are small membrane vesicles that shed from various cellular surfaces. Cellular microparticles expose membrane antigens that are specific for the cells from which they are derived and they vary in size, phospholipid and protein composition [Diamant et al., Eur J Clin Invest (2004) 34:392-401]. There are two mechanisms that can result in microparticle formation - cell activation and apoptosis. Endothelial cells produce microparticles when exposed to cytokines, such as interleukin-1 (IL-1) and tumor necrosis factor (TNF). Endothelial microparticles are detectable in normal human blood and are increased in patients with coagulation abnormalities. Concentration of circulating platelet microparticles (PMP) may serve as a marker of platelet activation. Procoagulant PMP are known to be elevated in severe thrombotic states [Preston et al., Hypertension (2003) 41:211-7]. Leukocyte-derived microparticles, bearing both tissue factor and P-Selectin glycoprotein ligand 1 (PSGL-1), circulate in blood and are accumulated in the developing platelet-rich thrombus following a vessel wall injury [Furie et al., Trends Mol Med (2004) 10:171-8].

A wide range of diseases demonstrate an increase in microparticles and are associated with procoagulability state. Endothelial microparticles are increased in patients with a coagulation abnormality associated with the lupus anticoagulant [Combes et al., J Clin Invest (1999) 104:93-102], and in acute coronary syndromes [Bernal-Mizrachi et al., Int J Cardiol (2004) 97:439-46]. Microparticles that shed from cancer cells constitute the main source TF activity and contribute to the prothrombotic effects associated cancer [Yu JL and Rak JW, J Thromb Haemost (2004) 2:2065-7]. In myeloproliferative syndromes, PMPs are elevated and provide a catalytic surface for thrombin generation that is associated with the increased risk for arterial or venous thrombotic event [Villmow et al., Thromb Res (2002) 108:139-45]. In patients with gastric cancer, PMP levels were significantly higher compared to healthy controls [Kanazawa et al., Lung Cancer (2003) 39:145-9]. Type 1 and Type 2 diabetes are also associated with increased levels of circulating microparticles. However, the procoagulant activity and the cellular origin of the microparticles differ in diabetic patients [Omoto et al., Nephron (1999) 81:271-7]. Normal pregnancies are characterized by high levels of platelet and endothelial microparticles compared to the levels of microparticles found in non-pregnant healthy women [Bretelle et al., Thromb Haemost (2003) 89:486-92].

Tissue Factor (TF), the initiator of coagulation, may appear in human plasma in a microparticle-associated or in a fluid-phase form. The microparticle associated forum is capable of initiating thrombin production (FVII-mediated), while the fluid phase form does not cause thrombin generation [Sturk-Maquelin et al., J Thromb Haemost (2003) 1(9):1920-6]. In order to maintain hemostatic balance and prevent hyper-coagulation, Tissue Factor Pathway Inhibitor (TFPI) inhibits the coagulation cascade by binding to factor VIIa/TF complex and to the active site of factor Xa consequently creating a quartet complex (TFPI/VIIa/TF/Xa). TFPI, which is found in circulating microparticles, was found to inhibit the enhanced TF activity in circulating MPs [Steppich et al., Thromb Haemost (2005) 93:35-9].

TF and TFPI antigens may also appear on intact cells, including endothelial cells and trophoblasts of the placenta, where they interact to maintain hemostatic balance. Endothelial cells possess an "anticoagulant character" and have been shown to express high TFPI protein levels and activity, while placental trophoblast cells possess a "procoagulant character" and have been shown to express high TF protein levels and activity [Aharon et al., Thromb Haemost (2004) 92:776-86].

At present, only a few commercially available coagulation assays and diagnostic kits exist. Screening tests, such as prothrombin time (PT) and activated partial thromboplastin time (aPTT), are most commonly used in clinical settings and measure the time it takes for a blood clot to form. The PT is the more convenient assay, and is performed by adding a large quantity of thromboplastin (TF) to the citrated plasma, with subsequent initiation of the reaction by calcium addition. The aPTT test involves a 3-5 minute preincubation of the citrated plasma with a mixture of phospholipids and negatively charged solid surfaces. The reaction is initiated by adding calcium. For both assays, the time to clot formation is evaluated. Although both assays are well established, neither assay entirely mimics the physiological coagulation reaction. For example, in the PT test, the source of TF utilized is thromboplastin and TF concentration is supraphysiological. This means that only the initiation phase of thrombin generation is required and the propagation and amplification phases are bypassed. The prothrombin time is therefore insensitive to many changes in the coagulation pathway and is incapable of detecting hypercoagulability [Fischer et al., U.S. Pat. No. 7,074,582].

Assays associated with the assessment of a hypercoagulable state include the Thrombin Anti-Thrombin Complex (TAT), Prothrombin fragment 1.2 (F1.2), and D-dimer. These blood tests are designed to measure a specific marker or product of the coagulation process. The TAT and F1.2 assays both measure late stages in the coagulation process while the D-dimer assay reflects both late stage of clot formation and fibrinolysis. These assays can exclude a thromboembolic disease but cannot foresee vascular complications or a risk of thrombotic event [Tejidor et al., U.S. Pat. No. 6,645,768].

U.S. Pat. No. 5,552,290 describes a method for detecting procoagulant platelet-derived microparticles (PDMP) in whole blood by flow cytometry. Total platelets are first identified using specific anti-platelet labeled agent (antibody against platelet-specific antigen, such as GPIb or GPIIb-IIIa).. Then procoagulant PDMP are identified with a second agent specific for procoagulant PDMP (antibodies directed against coagulant factors, such as coagulant factor II, V, VIII, or X, or a protein, such as Annexin V). This method enables assessment of only PDMPs and not of procoagulant microparticles from other cellular origins. Also, this method does not allow evaluation of tissue factor or TFPI expression on microparticles.

U.S. Pat. No. 7,005,271 discloses a method for determining a thrombotic or prethrombotic state of an individual. The method utilizes immunoassays for detection and characterization of circulating blood microparticles and stimulated procoagulant cells. Essentially, blood derived microparticles are immobilized on a solid phase and prothrombinase activity in the immobilized complex is determined. Elevated level of prothrombinase activity determined for the immobilized complex compared with a level determined for normal body fluid samples (e.g., blood) indicates a thrombotic or prethrombotic state. There is no indication for determining TFPI in the microparticles or for determining the ratio of TF to TFPI on the microparticles.

U.S. Pat. No. 20020076833 discloses the identification of blood coagulation and related medical conditions by analyzing an expression level of various markers in whole blood, platelets and microparticles. Although measurement of TF and TFPI is indicated, their specific expression on microparticles is not contemplated nor is determination of their ratio on such particles.

Shimura et al., (1996) discloses methods and kits for determing blood coagulation status of a blood sample comprising determining the TF to TFPI ratio in plasma.

Thus, currently, there is no coagulation assay that can measure TF or TFPI expression of particulated plasma and evaluate the expression ratio between particulated plasmatic TF and TFPI.

There is thus a widely recognized need for, and it would be highly advantageous to have a method of determining blood coagulation which is devoid of the above limitations.

### SUMMARY OF THE INVENTION

The invention is described by claims 1 to 8.

### BRIEF DESCRIPTION OF THE DRAWINGS

In the drawings:

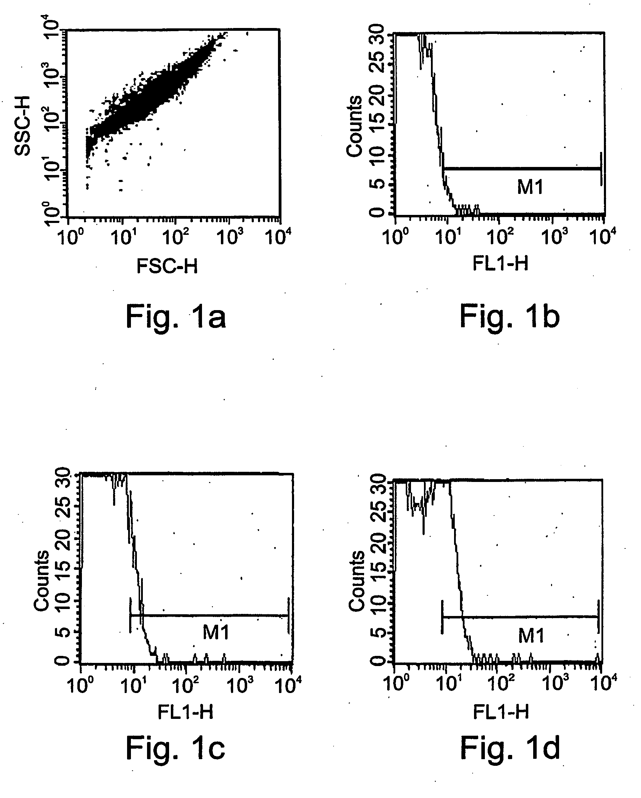

FIGs. 1A-D are graphs showing TF and TFPI expression on microparticles (MPs) obtained from a healthy non-pregnant female as determined by FACS. Figure 1A is a graph of the calibration beads - 0.75 micron presented in dot plot. Figure 1B is a graph showing anti-mouse FITC IgG-labeled sample which is used as a negative control. The M1 gate is indicated for the area of the labeled population (R1). Figure 1C is a graph showing TF labeled microparticles. M1 depicts TF-positive labeled microparticles. Figure 1D is a graph showing TFPI labeled microparticles. M1 depicts TFPI-positive labeled microparticles.

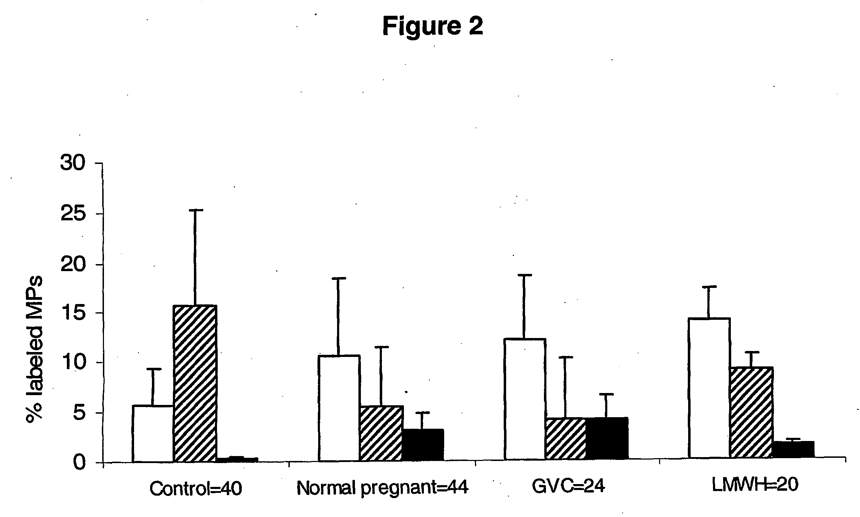

FIG 2 is a graph showing TF and TFPI expression and expression ratio on MPs in subjects with varied coagulation states as determined by FACS. The graph depicts TF or TFPI expression as percent of the total number of MPs in the gate. The TF/TFPI ratio reflects the coagulation status of the patient. Of note, open bars indicate TF expression, diagonal lines indicate TFPI expression and closed bars indicate TP/TFPI expression ratio. Shown are: TF and TFPI expression and expression ratio on MPs obtained from healthy non-pregnant women used as the control group (n=40); TF and TFPI expression and expression ratio on MPs obtained from healthy pregnant women indicated as normal pregnant (n=44); TF and TFPI expression and expression ratio on MPs obtained from pregnant women with gestational vascular complications (GVC, n=24); and TF and TFPI expression and expression ratio on MPs obtained from pregnant women with GVC treated with anticoagulant low molecular weight heparin (LMWH, n=20).

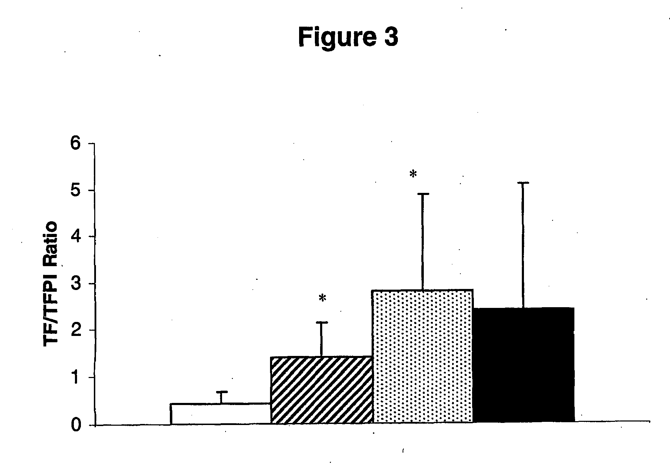

FIG. 3 is a graph showing TF/TFPI expression ratio on MPs in diabetic subjects as determined by FACS. MPs of healthy control volunteers (over 42 years of age, n=17, indicated by open bar), diabetic patients without known complications (n=13, indicated by diagonal lines bar), diabetic patients with cardio vascular complications (n=21, indicated by dotted bar) and diabetic patients with diabetic foot (n=22, indicated by closed bar).

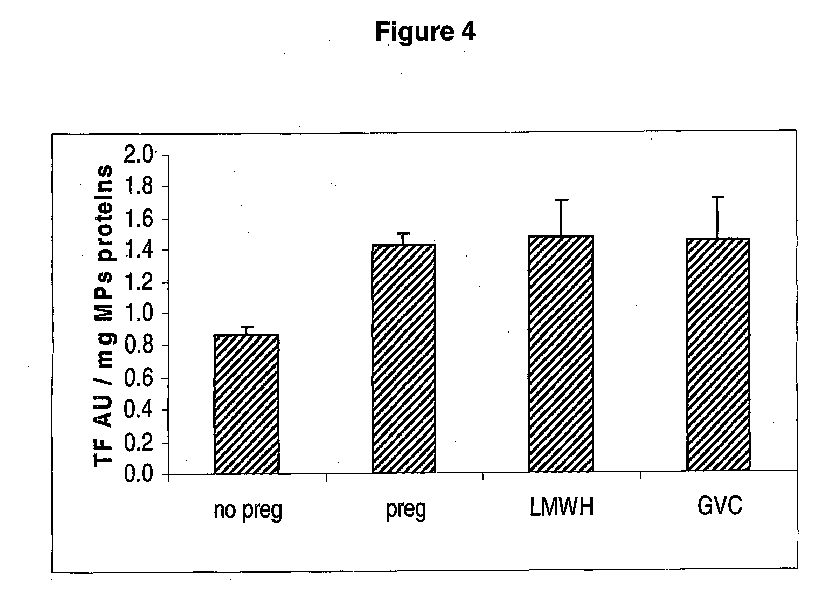

FIG. 4 is a graph showing TF activity on MPs in women subjects with varied coagulation states as measured by a one-step clotting assay. The clotting times were converted to standard curve of 10-1000 arbitrary units of TF (AU/ml) - where 180 seconds of clotting time stand for 1 TF AU, 130 seconds of clotting time stand for 1.5 TF AU and 100 seconds of clotting time stand for 2.5 TFAU. Shown are: TF activity on MPs obtained from healthy non-pregnant women used as the control group (n=7); TF activity on MPs obtained from healthy pregnant women indicated as pregnant (n=7); TF activity on MPs obtained from pregnant women with gestational vascular complications (GVC) treated with anticoagulant low molecular weight heparin (LMWH, n=7); and TF activity on MPs obtained from pregnant women with GVC (n=7).

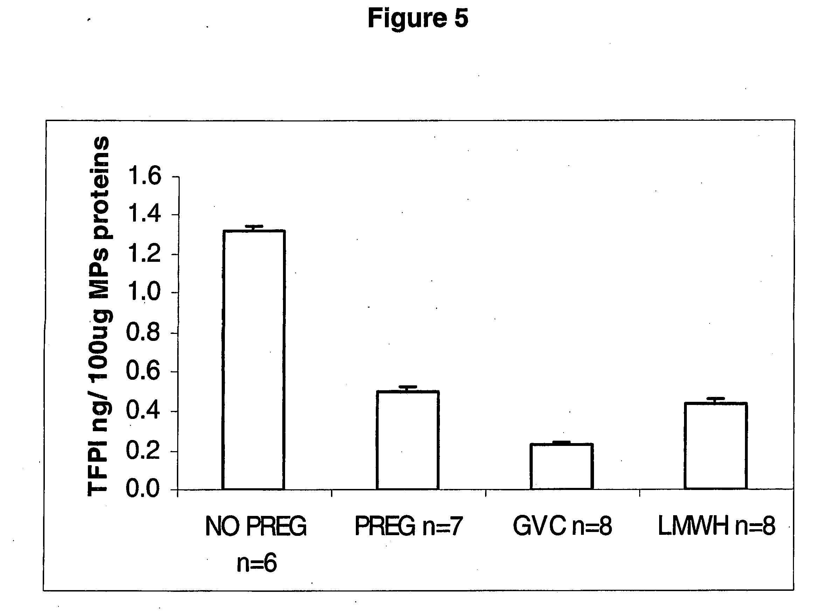

FIG 5 is a graph showing TFPI antigen levels on MPs in women subjects with varied coagulation states. TFPI protein levels were measured in human MPs extracts by ELISA and expressed as TFPI ng / 100 µg of MPs total proteins. Shown are: TFPI antigen level on MPs obtained from healthy non-pregnant women used as the control group (n=6); TFPI antigen level on MPs obtained from healthy pregnant women indicated as pregnant (n=7); TFPI antigen level on MPs obtained from pregnant women with gestational vascular complications (GVC, n=8); and TFPI antigen level on MPs obtained from pregnant women with GVC treated with anticoagulant low molecular weight heparin (LMWH, n=8):

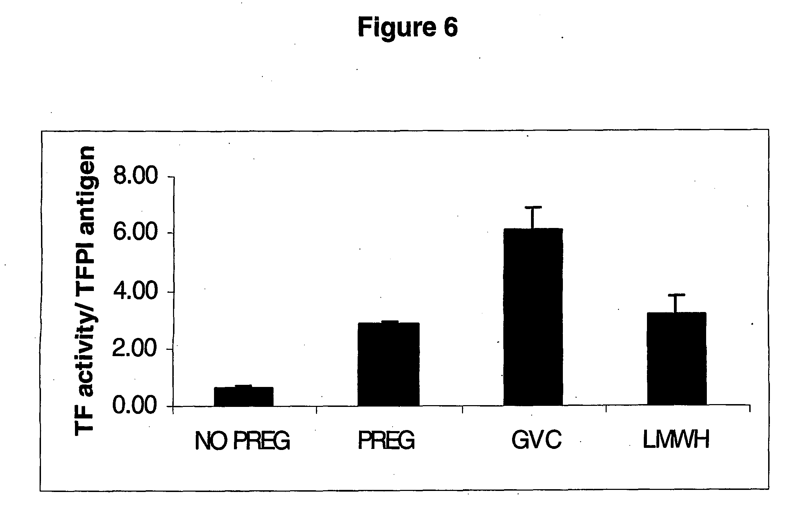

FIG 6 is a graph showing TF activity (shown in Figure 4) to TFPI antigen level (shown in Figure 5) ratio on MPs in women subjects with varied coagulation states. The TF activity/TFPI antigen ratio reflects the coagulation status of the patient. Shown are: TF activity/TFPI antigen ratio on MPs obtained from healthy non-pregnant women used as the control group (n=7); TF activity/TFPI antigen ratio on MPs obtained from healthy pregnant women indicated as pregnant (n=7); TF activity/TFPI antigen ratio on MPs obtained from pregnant women with gestational vascular complications (GVC, n=7); and TF activity/TFPI antigen ratio on MPs obtained from pregnant women with GVC treated with anticoagulant low molecular weight heparin (LMWH, n=7).

### DESCRIPTION OF THE PREFERRED EMBODIMENTS

The present invention is of a coagulation method which can be used for diagnosing the coagulation status of blood samples.

The principles and operation of the method according to the present invention may be better understood with reference to the drawings and accompanying descriptions.

Procoagulant microparticles are associated with changes in the coagulation balance and their quantity is highly elevated in a wide range of diseases including diabetes, cancer and acute coronary syndromes. Tissue Factor (TF), the initiator of coagulation, and Tissue Factor Pathway Inhibitor (TFPI), which inhibits the coagulation cascade, are both exposed as membrane antigens on the outer membrane of microparticles and influence the coagulation homeostasis.

At present, methods of assessing coagulation do not evaluate TF to TFPI expression ratio in microparticles. Coagulation assays and diagnostic kits available commercially are based on clotting time and do not entirely mimic the physiological coagulation reaction nor are able to detect hypercoagulability [Fischer et al., U.S Pat. No. 7,074,582].

U.S. Pat. No. 5,552,290 teaches a method of detecting procoagulant platelet-derived microparticles (PDMP) in whole blood by flow cytometry. This method is not appropriate for assessment of microparticles that are of other cellular origins and does not allow evaluation of TF or TFPI expression on microparticles.

U.S. Pat. No. 20020076833 teaches the identification of blood coagulation by analysis of expression levels of various markers in whole blood, platelets and microparticles. Although this method allows evaluation of TF and TFPI, it does not hint to their specific expression on microparticles nor their expression ratio.

U.S. Pat. No. 7,005,271 teaches characterization of prothrombinase activity in microparticles similarly to the aforementioned application. This method does not mention TFPI expression in microparticles nor determines TP to TFPI expression ratio in microparticles.

Whilst reducing the present invention to practice, the present inventors have discovered that TF to TFPI expression and/or activity ratio in cellular microparticles can be predictive of the blood coagulation status.

As is illustrated herein below and the Examples section which follows, the present inventors have revealed specific TF to TFPI ratios that can be predictive of blood coagulation status. For example, TF to TFPI expression ratio lower than 1 represents normal healthy human plasma (Example 1); TF to TFPI expression ratio above 1 demonstrates hypercoagulation; and TF to TFPI expression ratio higher than 3 may predict risk of vascular complications or thrombotic events (Examples 2 and 3).

These ratios were found predictive of hypercoagulation resultant of various conditions and disorders including pregnancy (Example 2), pregnancy associated with gestational vascular complications (GVC) (Example 2, Figure 2) and diabetes associated complications including cardio vascular conditions and diabetic foot (Example 3, Figure 3). These results conclusively show that the present teachings can be used for determining blood coagulation status in an accurate and simple manner.

Thus, according to one aspect of the present invention, there is provided a method of determining a coagulation status of a blood sample. The method comprising isolating cellular microparticles from the blood sample; and determining an expression and/or activity ratio of Tissue Factor (TF) to Tissue Factor Pathway Inhibitor (TFPI) in said cellular microparticles, wherein the expression ratio is indicative of the coagulation status of the blood sample.

As used herein, the phrase "coagulation status" refers to the coagulability of the blood which either provides hemostasis (also termed normal coagulation which refers to clot formation only at the site of vessel wall injury); displays an increased tendency for blood clotting and thromboembolism (hypercoagulation); or displays a bleeding tendency (hypocoagulation). Under physiological conditions, pro- and anti-coagulant mechanisms are delicately balanced to provide blood hemostasis. Disturbances in this balance result in either bleeding or thromboembolic disorders, and can be induced by medical conditions, congenital or acquired, or by the intake of drugs or vitamins.

As used herein, the phrase "blood sample" refers to a blood sample that contains cellular microparticles. According to a preferred embodiment of this aspect of the present invention, the blood sample may be fresh whole blood, fractionated whole blood, blood plasma and/ or microparticles.

As used herein, the phrase "cellular microparticles" refers to all blood cell derived microparticles. These microparticles are usually formed as a result of shedding (such as following cell activation, complement activity) and/or cell lysis (such as resulting from apoptosis). Examples of cellular microparticles which can be used in accordance with the present invention include, but are not limited to, platelet derived microparticles, endothelial cell derived microparticles, leukocyte derived microparticles and erythrocyte derived microparticles.

Blood withdrawal is effected using any procedure which is known in the art and provides enough material (cellular microparticles) for analysis. For example, normal venous blood collection procedures are used. Care is taken to not force blood from the subjects' veins. It is possible to use whole blood or plasma. It is especially preferred to use platelet poor plasma (PPP, see general materials and methods of the Examples section which follows). Preferably the sample is supplemented with an anticoagulant solution containing, for example, sodium citrate. The composition of the anticoagulant solution for collection of blood samples should keep platelet activation at a level as low as possible.

If needed microparticle enrichment may be effected by immobilizing this fraction to a solid support such as by immunoisolation using a microparticle specific antibody or antibodies. Accordingly, a concentration and separation of these microparticles from other blood cells and other blood or vascular compounds is possible. U.S. Pat. No. 7,005,271 provides detailed description for such an enrichment step. Any solid support may be used in such a configuration, preferably used are those which are compatible with automatic machinery such as multiwell plates, which can be used for high throughput analysis. Examples of such solid supports include, but are not limited to tubes, beads, microtiterplates or microcarriers made of plastics for example polystyrol, polyvinyl, polypropylene, polycarbonate, polysaccharide, silicone or glass [Maggio, Enzyme Immunoassays, CAP. Press, Florida (1980), 175-180; EP-A-0 063064, Bioengineering 16 (1974), 997-1003; Sonderson and Wilson, Immunology (1971) 20:1061-1065]. The microcarriers could be used as small columns.

As mentioned hereinabove, the expression and/or activity ratio of TF to TFPI in cellular microparticles of the blood sample is determined.

As used herein, the phrase "tissue factor (TF)" also termed thromboplastin, factor III or CD142 refers to the protein present as membrane receptor in subendothelial tissue, platelets, leukocytes and microparticles derived therefrom. TF is required for thrombin formation from the zymogen prothrombin, a stage which initiates the blood coagulation cascade.

As used herein, the phrase "tissue factor pathway inhibitor" refers to the single-chain polypeptide protein receptor which is present in platelets and endothelial cells. Tissue Factor Pathway Inhibitor (TFPI) regulates TF activity by inhibiting the FVIIa-TF complex and thus inhibits the coagulation cascade.

As used herein, the phrase "expression ratio" refers to the protein expression ratio of TF to TFPI in the above-described microparticles.

Determining TF to TFPI expression ratio in the microparticles can be effected using a homogeneous or heterogeneous assay. Homogeneous assays may be effected as described in U.S. Pat. No. 5,552,290. Heterogeneous assays refer to two phase assays, usually involving the immobilization of the microparticles on a solid support.

Determining TF to TFPI expression ratio at the protein level can be effected using methods which are well known in the art. Examples include, but are not limited to immunoassays, such as ELISA, FACS, Western blot and the like. As shown in Examples 1 and 5 of the Examples section which follows, TF to TFPI expression ratio was determined by FACS and TFPI antigen level was determined by ELISA.

As used herein, the phrase "activity ratio" refers to the ratio of TF activity to TFPI activity in the above-described microparticles.

As used herein "TF activity" refers to pro-coagulation activity which is TF dependent TF activity can be determined by assaying the clotting time of the microparticles using methods which are know in the art. For example, adding calcium and clotting factors (such as VIIa) to the microparticles and measuring clotting time (e.g., seconds) that may be converted to TF arbitrary units or the subsequent rate of factor Xa generation by chromogenic substrate.

As used herein "TFPI activity" refers to an anticoagulation activity which is TFPI dependent. TFPI is typically examined by a chromogenic activity assay. Basically, MPs are incubated with reagent mixture containing 0.8 nM activated factor X (Chromogenix-IL, Milan, Italy), 25 pM FVII (Sigma), 10 mM CaCl₂, 1 % TF (Innovin) in tris saline citrate buffer (0.05 M tris, 0.1 M NaCl, 0.01 M Na₃ Citrate, 0.2 % BSA (Sigma, pH8.0) for 20 minutes at 37 °C, followed by the addition of 0.4 U/ml FX (20 µl) and further incubation for 10 minutes. A chromogenic substrate for FXa - 0.72 mM S2765 (Chromogenix) is then added and incubated for 1 hour. The reaction is then terminated with 50 % acetic acid (50 µl). Absorbance is read at 405 nm and may be compared to a standard curve. TFPI activity may be expressed as percent inhibition of control.

The present inventors have uncovered that a hybrid ratio of TF activity (also referred to herein as functional TF) to TFPI expression (also referred to herein as TFPI antigen level) on microparticles can also be indicative of the coagulation status of the blood sample.

The present inventors have shown that TF to TFPI expression ratio of microparticles may be predictive of coagulation status. Thus, a TF to TFPI expression ratio lower than about 1 is indicative of normal coagulation; a TF to TFPI expression ratio higher than about 1 indicates hypercoagulation, while TF to TFPI expression ratio higher than about 3 may be indicative of a risk of vascular complications or thrombotic events. Similar results were obtained with the determination of functional TF to TFPI antigen level (i.e., expression) ratio as shown in Example 6.

Reagents (at least one) for determining expression and/or activity ratio of TF and TFPI in microparticles of blood samples may be included in a packaging kit.

Examples of such reagents may include antibodies directed at TF and TFPI (such markers are commercially available, see Examples section which follows).

According to a preferred embodiment of this aspect, the antibody comprises a label. The label can be an enzyme, chemiluminescent, fluorescent or any other label which will allow detection of the antibody. Alternatively, indirect labeling (e.g., secondary antibodies) may be used.

Examples of reagents which may be used for determining TFPI and TF activity include but are not limited to chromogenic substrate and coagulation factors such as described hereinabove.

As mentioned hereinabove, TF to TFPI expression ratio in microparticles may be predictive of a risk of hypercoagulation or prothrombosis. Thus, the present methodology may be valuable in determining treatment regimen for subjects in need thereof.

Thus, according to another aspect of the present invention, there is provided a method of designing a treatment regimen for a subject in need thereof. The method comprising, isolating cellular microparticles of a blood sample of the subject; determining on microparticles of a blood sample of the subject an expression and/or activity ratio of TF to TFPI as described above. The expression and/or activity ratio is indicative of the coagulation status of the subject; and designing the treatment regimen based on the coagulation status;

As used herein, the term "subject" refers to a mammalian subject, preferably a human subject. The subject may be at risk of hypercoagulation (e.g., predisposed) either because of a physiological state (e.g., pregnancy, a medical condition which affects, or results from abnormal blood coagulation), drug use or environmental or genetic predisposition.

Examples of medical conditions which affect or result from abnormal blood coagulation include, but are not limited to, disorders of the platelet and vessel wall [Immune thrombocytopenic purpura (ITP), Thrombotic thrombocytopenic purpura (TTP), Hemolytic-uremic syndrome (HUS), Glanzmann's thrombasthenia, Bernard-Soulier syndrome, Storage pool disorders, Paroxysmal nocturnal hemoglobinuria, Gray platelet syndrome: deficient alpha granules, Delta storage pool deficiency: deficient dense granules], disorders of coagulation and thrombosis [Disseminated intravascular coagulation, Factor deficiencies: Hemophilia A (Factor VIII deficiency), Hemophilia B (Factor IX deficiency, "Christmas disease"), Hemophilia C (Factor XI deficiency), Von Willebrand disease, Factor inhibitors, Platelet Dysfunction], disorders predisposing to thrombosis [Heparin-induced thrombocytopenia and thrombosis ("white clot syndrome"), Antiphospholipid syndrome, Lupus anticoagulant, Anticardiolipin antibody, Factor V Leiden, Activated Protein C Resistance, Prothrombin mutation, Protein C deficiency, Protein S deficiency, Antithrombin deficiency, Abnormally raised levels of Factor VIII and Factor XI], acute coronary syndromes, peripheral arterial diseases, diabetes mellitus, disseminated intravascular coagulation (DIC), cancer, systemic inflammatory diseases, atherosclerosis, thromboembolism, pulmonary embolism and pregnancy

As used herein, the phrase "treatment regimen" refers to a treatment plan that specifies the type of treatment, dosage, schedule and/or duration of a treatment provided to a subject in need thereof (e.g., a subject diagnosed with a pathology). The selected treatment regimen can be an aggressive one which is expected to result in the best clinical outcome (e.g., complete cure of the pathology) or a more moderate one which may relieve symptoms of the pathology yet results in incomplete cure of the pathology. It will be appreciated that in certain cases the more aggressive treatment regimen may be associated with some discomfort to the subject or adverse side effects (e.g., damage to healthy cells or tissue). The type of treatment can include a surgical intervention (e.g., removal of lesion, diseased cells, tissue, or organ), a cell replacement therapy, an administration of a therapeutic drug (e.g., receptor agonists, antagonists, hormones, chemotherapy agents) in a local or a systemic mode, an exposure to radiation therapy using an external source (e.g., external beam) and/or an internal source (e.g., brachytherapy) and/or any combination thereof. The dosage, schedule and duration of treatment can vary, depending on the severity of pathology and the selected type of treatment, and those of skills in the art are capable of adjusting the type of treatment with the dosage, schedule and duration of treatment.

Thus, once coagulation status is determined in accordance with the teachings of the present invention (either one time determination or repetitively, as necessary), the subject may be treated with, for example, blood thinners such as Low molecular weight heparins (LMWH), warfarin, aspirin, heparin, NSAIDs, Dipyridamole, Clopidogrel and Plateles glycoprotein IIb/IIIa antagonists.

Thus, the present invention provides novel methods and kits for determining blood coagulation that can be employed for designing treatment regimen for numerous subjects in need thereof.

As used herein the term "about" refers to ± 10 %.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996)

### GENERAL MATERIALS AND METHODS

***Blood collection and preparation:*** The study was approved by the ethics comity of the Rambam Health Care Campus. 3 ml blood samples were collected into blood collection tubes containing 300 µl Sodium Citrate (1:10). Tubes were centrifuged twice at 1,500 x g for 15 minutes in order to reach Poor-Platelet Plasma (PPP) state. The samples were then used in the assay kit or were stored for up to one week in a -80 °C freezer.

***Antibodies:*** The following antibodies were used: mouse anti-human TF (American Diagnostica, Greenwich, CT, USA); FITC conjugated mouse anti-human TF (American Diagnostica, Greenwich, CT, USA); mouse anti-human TFPI (American Diagnostica, Greenwich, CT, USA); rabbit anti mouse IgG FITC (DakoCytomation, Denmark); FITC conjugated mouse IgG (BD biosciences, Fremingham, MA, USA) and Beads 0.75 micron (BD biosciences, Fremingham, MA, USA).

***Sample labeling and FACS analysis:*** Tubes containing 50 µl sample PPP were incubated for 30 minutes at room temperature (RT) with either FITC conjugated mouse anti-human TF antibody or FITC conjugated mouse anti-human TFPI antibody as primary antibodies. FITC conjugated mouse IgG was used as a secondary antibody and samples which were stained only with FITC conjugated mouse IgG were used as a negative control.

Samples stained with non conjugated antibodies, TF or TFPI, for 30 minutes at RT were then incubated with secondary antibody FITC anti mouse for 30 minutes, in the dark.

450 µl FACS buffer (Phosphate buffer saline (PBS), Formaldehyde 1 %, Azid 0.02 %) was added to each tube.

All particles were identified by FACS analysis and are shown in Figure 1A. TF labeled microparticles and TFPI labeled microparticles were identified at the M1 area (in Figures 1B, D) and expressed as percent from the total microparticles in the gate.

***Calculation of TF*/*TFPI ratio:*** The ratio between TF and TFPI expression on microparticles was calculated as a measure of coagulation status. TF/TFPI ratio less than 1 represented normal healthy human plasma. TF/TFPI ratio higher than 1 demonstrated hyper-coagulability state. TF/TFPI ratio higher than 3 may predict a risk of vascular complication or a thrombotic event.

***TF activity:*** Plasmatic MPs were isolated using ultracentrifuge. MPs µg/µl TF activity was measured by a one-step clotting assay as was previously described (Aharon et al, Thromb Haemost (2004) 92(4):776-86). MPs extract. (100 µl) were added to pooled normal human plasma (100 µl) and incubated for one minute at 37°C. Then, a 25 mM calcium chloride solution (100 µl) was added. The clotting times were measured and converted to standard curve of 10-1000 arbitrary units of TF (AU/ml) - where 180 seconds of clotting time stand for 1 TF AU, 130 seconds stand for 1.5 TF AU and 100 sec stand for 2.5 TF AU.

***Measurement of TFPI antigen levels by ELISA**:* Plasmatic MPs were isolated using ultracentrifuge. TFPI protein levels were measured in human MPs extracts by ELISA (American Diagnostica, Greenwich, CT, USA) according to manufacturer's instructions and expressed as TFPI ng/100 µg of MPs total proteins.

***Calculation of TF activity*/*TFPI antigen level ratio:*** The ratio between TF activity and TFPI antigen level on microparticles was calculated as a measure of coagulation status. TF activity/TFPI. antigen ratio less than 1 represented normal healthy human plasma. TF activity/TFPI antigen ratio higher than 1 demonstrated hyper-coagulability state. TF activity/TFPI antigen ratio higher than 3 may predict a risk of vascular complication or a thrombotic event.

### EXAMPLE 1

### Assessing the ratio between TF and TFPI expression on microparticles as the method of the present invention

### RESULTS

To characterize the normal TF/TFPI ratio of blood microparticles (MPs), a blood sample of a healthy non-pregnant woman was analyzed for MPs TF and TFPI expression. FACS analysis was used to identify positively stained MPs. FACS mediated detection of MPs was calibrated with 0.75 micron beads as indicated in Figure 1A. Sample labeling with anti mouse FITC IgG served as the negative control (Figure 1B). The ratio between TF expression (6.4%, Figure 1C) and TFPI expression (18.5%, Figure 1D) on microparticles was calculated (0.35). The TF/TFPI ratio was lower than 1 which represented normal healthy human plasma.

### EXAMPLE 2

### Determining TF and TFPI expression ratio on MPs of women as a method to assess hyper-coagulation and pro-thrombotic events in pregnancy according to the teachings of the present invention

### RESULTS

Four groups of women: non-pregnant healthy women (control group, n=40), healthy pregnant women (normal pregnant, n=44), pregnant women with gestational vascular complications (GVC, n=24) and pregnant women with GVC treated with anticoagulant low molecular weight heparin (LMWH, n=20) were examined to characterize the thrombogenic potential of microparticles. The expression of TF or TFPI and their ratio on blood microparticles was assessed.

As shown in Figure 2, expression of TF on microparticles was significantly higher in pregnant women compared to non-pregnant women aged 22-40 years old (p=0.0002). There was no marked difference between TF expression in the three different pregnant women groups (healthy, with GVC or with GVC treated with LMWH).

The results also demonstrate that expression of TFPI on microparticles was relatively high in non-pregnant women whereas it was significantly lower in normal pregnant women (p<0.0001). Expression of TFPI was even lower in pregnant women with GVC (p=0.41) a value which rose significantly in pregnant women with GVC treated with enoxaparin (LMWH, p=0.042).

The microparticle TF/TFPI ratio in non-pregnant women was 0.396 (±0.195). The ratio increased significantly to 2.78 (±0.124, p=0.0092) in normal pregnant women and increased even more significantly to 4.17 (±2.32, p=0.0092) in pregnant women with GVC. The microparticle TF/TFPI ratio was significantly reduced in the LMWH treated group 1.58 (±0.437, p=0.0001) compared to non-treated GVC pregnant women.

The results demonstrated that microparticle TF/TFPI ratio was lower than 1 in non-pregnant healthy woman as expected in normal healthy human plasma. In healthy pregnant women, the microparticle TF/TFPI ratio was higher than 1 which implies a hyper-coagulability state. In pregnant women with known gestational vascular complications, the microparticle TF/TFPI ratio was higher than 3 which may predict a thrombotic event. The TF/TFPI ratio was significantly reduced in the LMWH treated group.

### EXAMPLE 3

### Determining TF and TFPI expression ratio on MPs of subjects with diabetes as a method to assess hyper-coagulation and pro-thrombotic events according to the teachings of the present invention

### RESULTS

To characterize the thrombogenic potential of microparticles, blood was obtained from healthy volunteers (control group, n=17), diabetic healthy patients (n=13), diabetic patients with cardiovascular complications (n=21) and patients with diabetic foot (n=22). The expression of TF and TFPI on microparticles and their ratio was assessed.

As shown in Figure 3, the TF/TFPI ratio on microparticles of the healthy control group was 0.43 (±0.268) and was significantly increased in diabetic patients without known complications 1.41 (±0.734, p>0.0001). The TF/TFPI ratio was further increased in diabetic patients with cardio vascular complications 2.8 (±2.08, p=0.028) or with diabetic foot 2.38 (±2.7, p=0.021).

The results demonstrated that microparticle TF/TFPI ratio was lower than 1 in the healthy control group as expected in normal healthy human plasma. The microparticle TF/TFPI ratio increased to higher than 1 in all diabetic patients, with even higher ratio values in diabetic patients with diabetic foot, which implies a hyper-coagulability state. In diabetic patients with cardio vascular complications the microparticle TF/TFPI ratio may predict a thrombotic event.

### EXAMPLE 4

### Determining MP TF activity in women population groups

### RESULTS

Four groups of women: non-pregnant healthy women (control group, n=7), healthy pregnant women (pregnant, n=7), pregnant women with gestational vascular complications (GVC, n=7) and pregnant women with GVC treated with anticoagulant low molecular weight heparin (LMWH, n=7) were examined to characterize the procoagulant potential of microparticles. TF activity on blood microparticles was assessed.

As demonstrated in Figure 4, TF activity on microparticles was significantly lower in non-pregnant women compared to pregnant women (p<0.0001). There was no marked difference between TF activity in the three different pregnant women groups (healthy, with GVC treated with LMWH or untreated with GVC).

### EXAMPLE 5

### Determining MP TFPI antigen levels in women population groups

### RESULTS

Four groups of women: non-pregnant healthy women (control group, n=6), healthy pregnant women (pregnant, n=7), pregnant women with gestational vascular complications (GVC, n=8) and pregnant women with GVC treated with anticoagulant low molecular weight heparin (LMWH, n=8) were examined to characterize the anticoagulant potential of microparticles. TFPI antigen levels on blood microparticles were assessed.

As demonstrated in Figure 5, TFPI antigen level on microparticles of non-pregnant healthy women was significantly higher compared to pregnant healthy women 1.322±0.0258; 0.504±0.0161 respectively (p<0.0001). TFPI antigen level was even lower in pregnant women with GVC 0.236±0.013 (p<0.0001), a value which rose in pregnant women with GVC treated with LMWH 0.445± 0.019 (p<0.0001).

### EXAMPLE 6

### Determining TF activity and TFPI antigen level ratio in MPs of women as a method to assess hyper-coagulation and risk of pro-thrombotic events in pregnancy according to the teachings of the present invention

### RESULTS

To characterize the thrombogenic potential of microparticles, blood was obtained from healthy non-pregnant women (control group, n=7), healthy pregnant women (pregnant, n=7), pregnant women with gestational vascular complications (GVC, n=7) and pregnant women with GVC treated with anticoagulant low molecular weight heparin (LMWH, n=7). TF activity to TFPI antigen level on blood microparticles was assessed.

As shown in Figure 6, TF activity/TFPI antigen ratio on microparticles of non-pregnant women was 0.657±0.035. The ratio increased significantly to 2.82±0.135 (p<0.0001) in healthy pregnant women and increased drastically to 6.135±0.768 (p<0.0001) in pregnant women with GVC. The microparticle TF activity/TFPI antigen ratio was significantly reduced in the LMWH treated group to 3.198 ±0.568 (p<0.0001) compared to non-treated GVC pregnant women.

The results demonstrated that microparticle TF activity/TFPI antigen ratio was lower than 1 in non-pregnant healthy woman as expected in normal healthy human plasma. In healthy pregnant women, the microparticle TF activity/TFPI antigen ratio was higher than 1 which implies a hyper-coagulability state. In pregnant women with known gestational vascular complications, the microparticle TF activity/TFPI antigen ratio was higher than 3 which may predict a risk of thrombotic event. The TF activity/TFPI antigen ratio was significantly reduced in the LMWH treated group.

## Claims

1. A method of determining a coagulation status of a blood sample, the method comprising;
(a) isolating cellular microparticles from the blood sample; and
(b) determining an expression and/or activity ratio of Tissue Factor (TF) to Tissue Factor Pathway Inhibitor (TFPI) in said cellular microparticles, wherein said ratio is indicative of the coagulation status of the blood sample.

2. A method of designing a treatment regimen for a subject in need thereof, the method comprising:
(a) isolating cellular microparticles from a blood sample of the subject;
(b) determining on cellular microparticles of a blood sample of the subject an expression and/or activity ratio of TF to TFP1, wherein said expression and/or activity ratio is indicative of the coagulation status of the subject; and
(c) designing the treatment regimen based on said coagulation status.

3. The method of claim 2, wherein the treatment is selected from the group consisting of Low molecular weight heparins (LMWH), warfarin, aspirin, heparin, NSAIDs, Dipyridamole, Clopidogrel and Plateles glycoprotein IIb/IIIa antagonists.

4. The method of claim 1 or 2, wherein said cellular microparticles are selected from the group consisting of platelet derived microparticles, endothelial cell derived microparticles, leukocyte derived microparticles and erythrocyte derived microparticles.

5. The method of claim 1 or 2, wherein said activity ratio is determined by a clotting assay.

6. The method of claim 1 or 2, wherein when said expression ratio of TF to TFPI is below about 1, the coagulation status of the blood sample is normal.

7. The method of claim 1 or 2, wherein when said expression ratio of TF to TFPI is above about 1 the coagulation status of the blood sample demonstrates hyper-coagulability.

8. The method of claim 1 or 2, wherein when said expression ratio of TF to TFPI is above about 3, the coagulation status of the blood sample is predictive of the risk for thrombotic events.

## Patentansprüche

1. Verfahren zum Bestimmen eines Gerinnungszustands einer Blutprobe, wobei das Verfahren umfasst:
(a) Isolieren von zellulären Mikropartikeln aus der Blutprobe; und
(b) Bestimmen eines Expression- und/oder Aktivitätsverhältnisses von Gewebefaktor (TF) zu Gewebefaktor-Inhibitor (TFPI) in den zellulären Mikropartikeln, wobei das Verhältnis für den Gerinnungszustand der Blutprobe bezeichnend ist.

2. Verfahren zum Entwerfen eines Behandlungsregimes für einen Probanden mit Bedarf daran, wobei das Verfahren umfasst:
(a) Isolieren von zellulären Mikropartikeln aus einer Blutprobe des Probanden;
(b) Bestimmen bei zellulären Mikropartikeln einer Blutprobe des Probanden eines Expressions- und/oder Aktivitätsverhältnisses von TF zu TFPI, wobei das Expressions- und/oder Aktivitätsverhältnis für den Gerinnungszustand des Probanden bezeichnend sind; und
(c) Entwerfen des Behandlungsregimes basierend auf dem Gerinnungszustand.

3. Verfahren nach Anspruch 2, wobei die Behandlung aus der Gruppe bestehend aus niedermolekularem Heparin (LMWH), Warfarin, Aspirin, Heparin, NSAIDs, Dipyridamol, Clopidogrel und Blutplättchen-Glycoprotein IIb/IIIa-Antagonisten ausgewählt ist.

4. Verfahren nach Anspruch 1 oder 2, wobei die zellulären Mikropartikel aus der Gruppe bestehend aus von Blutplättchen gewonnenen Mikropartikeln, von Endothelzellen gewonnenen Mikropartikeln, von Leukozyten gewonnenen Mikropartikeln und von Erythrozyten gewonnenen Mikropartikeln ausgewählt sind.

5. Verfahren nach Anspruch 1 oder 2, wobei das Aktivitätsverhältnis durch einen Gerinnungstest bestimmt wird.

6. Verfahren nach Anspruch 1 oder 2, wobei, wenn das Expressionsverhältnis von TF zu TFPI unter etwa 1 liegt, der Gerinnungszustand der Blutprobe normal ist.

7. Verfahren nach Anspruch 1 oder 2, wobei, wenn das Expressionsverhältnis von TF zu TFPI über etwa 1 liegt, der Gerinnungszustand der Blutprobe Hyperkoagulabilität zeigt.

8. Verfahren nach Anspruch 1 oder 2, wobei, wenn das Expressionsverhältnis von TF zu TFPI über etwa 3 liegt, der Gerinnungszustand der Blutprobe für die Gefahr von thrombotischen Ereignissen prädiktiv ist.

## Revendications

1. Procédé de détermination d'un état de coagulation dans un échantillon de sang, le procédé comprenant les étapes consistant à :
(a) isoler des microparticules cellulaires de l'échantillon de sang ; et
(b) déterminer un rapport d'expression et/ou d'activité entre le facteur de tissu (TF) et l'inhibiteur de la voie du facteur de tissu (TFPI) dans lesdites microparticules cellulaires, où ledit rapport est indicatif de l'état de coagulation de l'échantillon de sang.

2. Procédé de conception d'un régime de traitement pour un sujet qui en a besoin, le procédé comprenant les étapes consistant à :
a) isoler des microparticules cellulaires d'un échantillon de sang du sujet ;
(b) déterminer sur les microparticules cellulaires d'un échantillon de sang du sujet un rapport d'expression et/ou d'activité entre TF et TFPI, où ledit rapport d'expression et/ou d'activité est indicatif de l'état de coagulation du sujet ;
(c) concevoir le régime de traitement en se basant sur ledit état de coagulation.

3. Procédé selon la revendication 2, dans lequel le traitement est choisi dans le groupe consistant en les héparines de faible masse moléculaire (LMWH), la warfarine, l'aspirine, l'héparine, les AINS, le dipyridamole, le clopidogrel et les antagonistes de glycoprotéine IIb/IIIa des plaquettes.

4. Procédé selon la revendication 1 ou 2, dans lequel lesdites microparticules cellulaires sont choisies dans le groupe consistant en les microparticules dérivées des plaquettes, les microparticules dérivées de cellules endothéliales, les microparticules dérivées de leucocytes et les microparticles dérivées d'érythrocytes.

5. Procédé selon la revendication ou 2, dans lequel ledit rapport d'activité est déterminé par un dosage de coagulation.

6. Procédé selon la revendication ou 2, dans lequel lorsque ledit rapport d'expression entre TF et TFPI est inférieur à environ 1, l'état de coagulation de l'échantillon de sang est normal.

7. Procédé selon la revendication 1 ou 2, dans lequel lorsque ledit rapport d'expression entre TF et TFPI est au-dessus d'environ 1, l'état de coagulation de l'échantillon de sang manifeste une hypercoagulabilité.

8. Procédé selon la revendication 1 ou 2, dans lequel lorsque ledit rapport d'expression entre TF et TFPI est au-dessus d'environ 3, l'état de coagulation de l'échantillon de sang est prédictif du risque de survenue d'événements thrombotiques.
